**Europäisches Patentamt**

**European Patent Office**

**Office européen des brevets**

(19)

(11) Publication number: **0 228 178**
**B1**

(12) ## EUROPEAN PATENT SPECIFICATION

(45) Date of publication of the patent specification:
**03.10.90**

(51) Int. Cl.⁵: **A61B 7/04, A61B 5/103, H04R 11/00**

(21) Application number: **86308971.0**

(22) Date of filing: **18.11.86**

(54) **Displacement sensor.**

(30) Priority: **27.12.85 JP 294650/85**
**21.02.86 JP 24383/86 U**

(43) Date of publication of application:
**08.07.87 Bulletin 87/28**

(45) Publication of the grant of the patent:
**03.10.90 Bulletin 90/40**

(84) Designated Contracting States:
**CH DE FR GB LI**

(56) References cited:
**GB-A- 1 205 569**
**GB-A- 2 095 072**
**US-A- 3 790 712**
**US-A- 4 071 694**

**PATENT ABSTRACTS OF JAPAN, vol. 7, no. 258 (E-211)[1403], 17th November 1983; & JP-A-58 143 697 (MITACHI ONKIYOU SEISAKUSHO K.K.) 26-08-1983**

(73) Proprietor: **TDK Corporation, 13-1, Nihonbashi 1-chome Chuo-Ku, Tokyo-to(JP)**

(72) Inventor: **Mohri, Kaneo, 1-4-24, Yayoi Sawara-ku, Fukuoka-shi Fukuoka(JP)**

(74) Representative: **Rackham, Stephen Neil et al, GILL JENNINGS & EVERY 53-64 Chancery Lane, London WC2A 1HN(GB)**

## Description

This invention relates to a displacement sensor and, in particular, to a displacement sensor which senses displacement or vibration of a living body. The invention is particulary applicable to a stethoscope for medical purposes, or to a mechanocardiograph.

Conventionally, acoustic stethoscopes have been used for examining living bodies. Prior stethoscopes comprise a diaphragm which is placed on a surface of a living body, so that the vibration of the diaphragm can be listened to by a doctor through a stethoscope tube.

The prior stethoscopes have the disadvantage that only a single doctor can listen to the sound of the vibration of heart valves in a living body, and the sound cannot be listened to by a plurality of persons simultaneously. Furthermore, prior stethoscopes have the disadvantage that a doctor cannot listen to the sound of heart beats occurring at a low frequency, for example below 20 Hz. Also, the output of the stethoscope cannot be recorded.

On the other hand, prior electrocardiographs record pulsation or drive electrical signals of the heart, but do not record actual mechanical movements of the heart. Therefore, if there is something wrong with the heart such as a valve disease or arteriosclerosis, this could not be detected by a prior electrocardiograph. Conventional mechanocardiograph sensors for detection of heart movement are difficult to operate.

US-A 3 790 712 discloses a stethoscope that includes a microphone to detect sounds. The sounds are converted into electrical singnals and then relayed to one or more headsets befor conversion back to acoustic signals by a transducer. In this way the detected sounds are simultaneously available to more than one listener. US-A 4 071 694 discloses a stethoscope that can be used in either an acoustic or an electronic mode. Again the stethoscope includes a microphone but is also directly acoustically coupled to the earpieces.

According to the invention there is provided a displacement sensor having a diaphragm which is to be placed on a moving body; a magnetic pole movable with the diaphragm; a plurality of inductors located radially around the magnetic pole on a plane which is perpendicular to the direction of movement of the diaphragm to provide an output signal in dependence upon the position of the magnetic pole, the inductors being divided into two groups of alternate inductors with the inductors in each group being connected in series; and a processor for processing the output signal of the incutors.

Preferably the processor comprises an astable multivibrator. Each of the inductors preferably comprises a plurality of series-conneced inductance elements arranged in a star shape so that the earth's magnetism and undesired magnetic noise are cancelled.

An embodiment of the invention will now be described, by way of example, with reference to the accompanying drawings, wherein

Fig. 1 is a partly exploded view of a displacement sensor according to the invention;

Fig. 2A is a plan view of a magnetic head of the displacement sensor of Fig. 1;

Fig. 2B is a cross section on a line A-A' of Fig. 2A;

Fig. 3 shows the electrical interconnection of inductors of the magnetic head of Fig. 2A;

Fig. 4 is a block diagram of a processor used in the displacement sensor of Fig. 1;

Figs. 5 and 6 are experimental curves showing characteristics of a displacement sensor according to the invention;

Figs. 7, 8 and 9 show cardiograms obtained from a living body using the displacement sensor; and

Fig. 10 is a curve showing an output voltage/displacement characteristic of the displacement sensor.

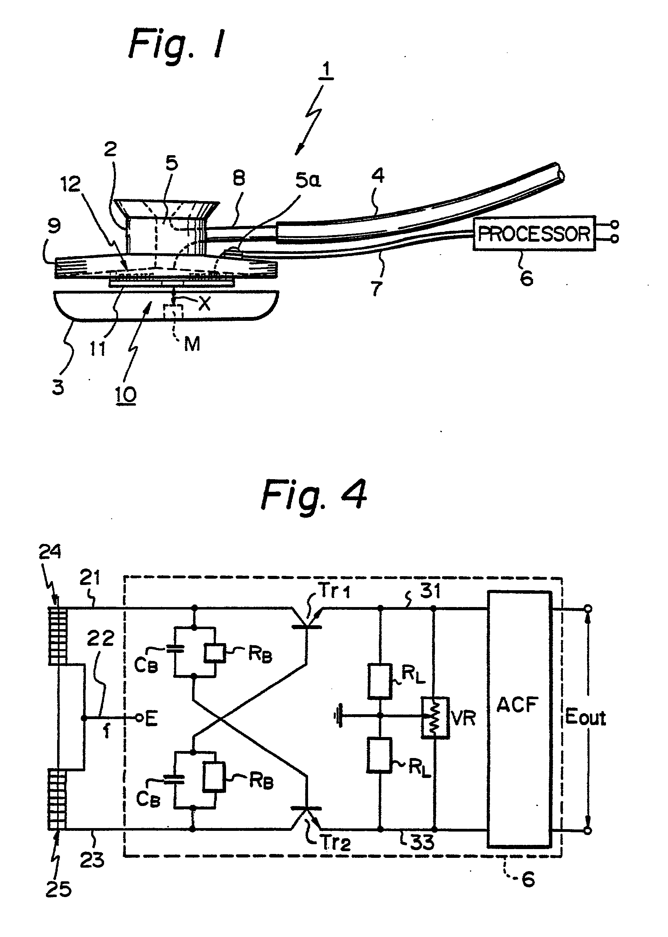

Fig. 1 shows a structure of a displacement sensor or stethoscope 1 having a main body 2, a diaphragm 3 and a magnetic head 10.

A rigid tube 8 is connected to the main body 2, and a flexible tube 4 is connected to the tube 8. Accordingly, an acoustic vibration set up in a space 5 in the main body 2 propagates through the rigid tube 8 to the flexible tube 4, the free end of which can be applied to an ear of a doctor. The main body 2 has a screw thread 9 around its periphery and the edge of the diaphragm 3 engages with that screw thread. The magnetic head 10 is mounted between the diaphragm 3 and the main body 2.

The head 10 functions to generate an electrical signal according to vibrations of the diaphragm 3. The head comprises a magnetic pole M (a small permanent magnet) coupled to the diaphragm, a group of inductors 12 the inductance of which varies according to the displacement of the magnetic pole M, and a hole 11 for mounting the inductors 12. An output terminal block 5a is mounted on the main body 2, so that lead-out wires of the inductors 12 are coupled to a sensor cable 7 via the terminal block. The far end of the sensor cable 7 is coupled to a processor 6 which processes the signal from the head 10.

Accordingly, the stethoscope of Fig. 1 functions both as a conventional stethoscope which propagates acoustic vibrations to an ear, and as an electric stethoscope which provides electrical output information according to vibrations of the diaphragm 3.

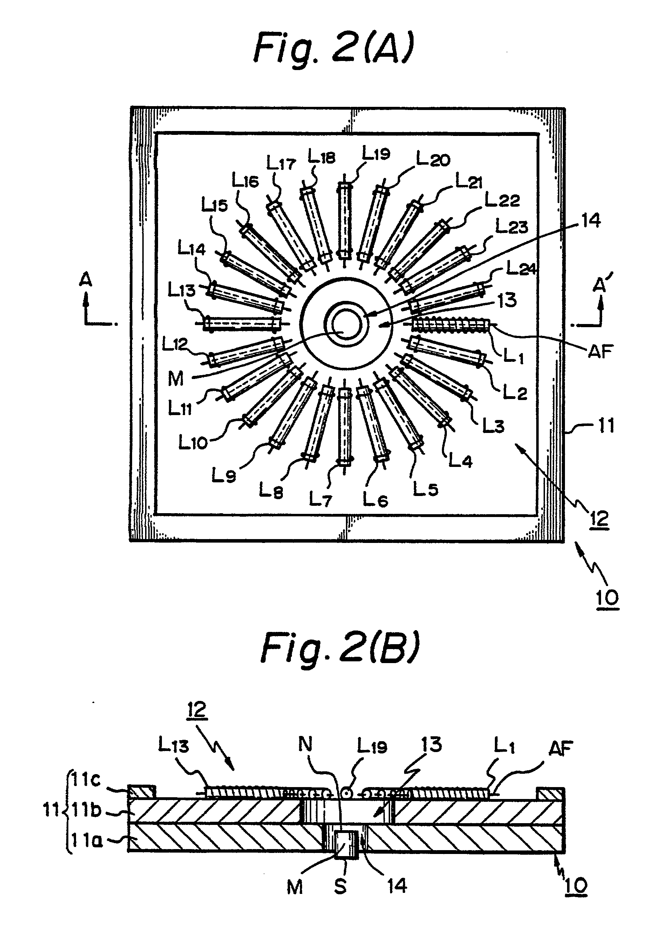

Fig. 2A shows a plan view of the head 10, and Fig. 2B shows a cross section of the head. The magnet M mounted on the diaphragm 3 is, in the present embodiment, a cylindrical permanent magnet of 3 mm diameter. The holder 11 comprises a first insulating member 11a, a second insulating member 11b and a third insulating member 11c, laminated together. The member 11a has a circular hole 14 at its centre so that the permanent magnet M is movably received in the hole. The member 11b also has a central hole 13, which is coaxial with the hole 14 and is slightly larger than the hole 14. he group of inductors 12 is positioned radially around the hole 13. The member 11c is located at the periphery of the member 11b so that

the member 11c provides a space for mounting the inductors 12.

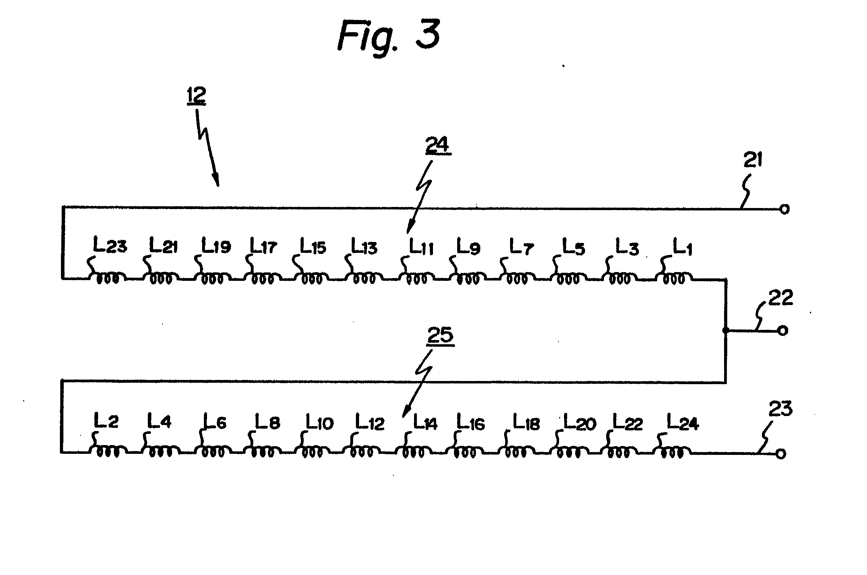

Each of the saturable inductors 12 ($L_1$ to $L_{24}$) comprises a core wire AF and a coil wound on the wire AF. The wire AF is, for instance, made of amorphous material, the composition of which is, for example, $Co_{68}Fe_4Si_{13}B_{15}$ in atomic percent. The diameter of the wire AF is, for instance, 110μm and its length is 3 mm. Referring also to Fig. 3, the coils are connected in series alternately so that the coils $L_1$, $L_3$, $L_5$, $L_7$, $L_9$, $L_{11}$, $L_{13}$, $L_{15}$, $L_{17}$, $L_{19}$, $L_{21}$ and $L_{23}$ are connected in series, and the coils $L_2$, $L_4$, $L_6$, $L_8$, $L_{10}$, $L_{12}$, $L_{14}$, $L_{16}$, $L_{18}$, $L_{20}$, $L_{22}$ and $L_{24}$ are connected in series. The first inductor group $L_1$ to $L_{23}$ and the second inductor group $L_2$ to $L_{24}$ are connected in series by connecting one end of the coil $L_1$ to one end of the coil $L_2$. Lead-out wires 21, 22 and 23 for coupling to an external circuit are provided at one end of the coil $L_{23}$, the junction between the coils $L_1$ and $L_2$, and one end of the coil $L_{24}$, respectively. The wires 21, 22 and 23 are connected to the processor 6 through the sensor cable 7.

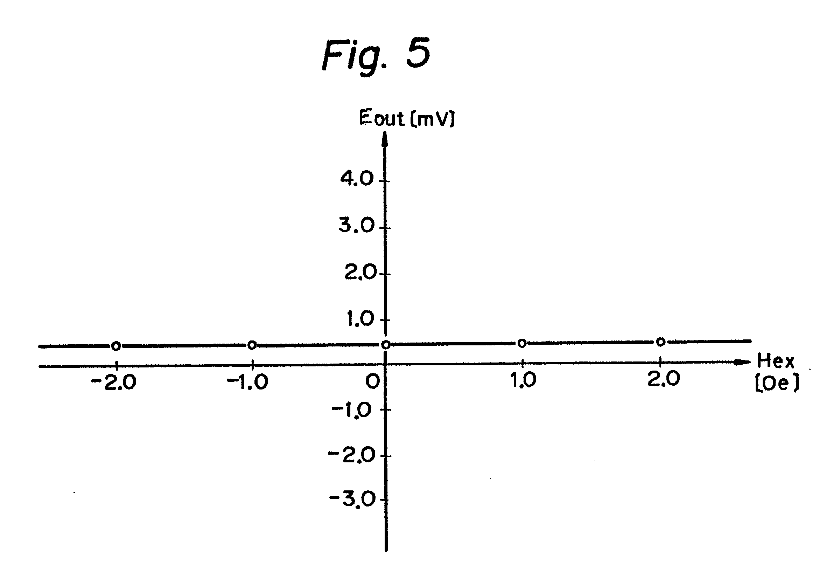

Fig. 4 is a block diagram of the processor 6, which includes an astable multivibrator and an active filter. In Fig. 4 the numerals 24 and 25 indicate the inductor group $L_1$ to $L_{23}$ and the inductor group $L_2$ to $L_{24}$, respectively. The structure of the processor 6 is basically an astable multivibrator circuit comprising a pair of transistors Tr1 and Tr2, the base of each of which is coupled to the collector of the other transistor through a respective parallel circuit of a capacitor $C_B$ and a resistor $R_B$. The collector of the transistor Tr1 is connected to the lead-out wire 21, and the collector of the transistor Tr2 is connected to the lead-out wire 23. The emitters of the transistors Tr1 and Tr2 are coupled to an active filter ACF via lines 31 and 33, respectively. The emitters are bridged by a series circuit of a pair of load resistors $R_L$, and a variable resistor VR. The junction point of the load resistors $R_L$ and the slider of the variable resistor VR are grounded. The variable resistor VR adjusts the state of balance of a bridge circuit comprising the inductance groups 24 and 25 and the two resistors $R_L$ so that an output signal $E_{out}$ of the filter ACF is zero when the diaphragm 3 does not vibrate. The output signal $E_{out}$ is the output of the processor 6.

The first group 24 of inductors and the second group 25 of inductors are connected in series so that a differential output of the inductors is fed to the wires 21 and 23. The junction point of the groups of coils (lead-out wire 22) is coupled to a power source E. The frequency of oscillation f of the multivibrator is in the range between 100kHz and 500 kHz and is preferably 200kHz. The higher the frequency f is, the more advantageous it is, to induce a higher voltage. The upper limit of the frequency f is determined by the operational characteristics of the amorphous material of which the coils are made.

Preferably, the characteristics of the active filter are adjustable by switching. The active filter may be (1) a low-pass filter with a cut-off frequency of 600Hz, (2) a low-pass filter with a cut-off frequency of 20Hz, (3) a high-pass filter with a cut-off frequency of 20Hz, or (4) a bandpass filter which passes a band from DC to 20Hz or a band from 20Hz to 600Hz.

When the two groups of coils 24 and 25 are excited by the signal of frequency f, the output level $E_{out}$ is zero if the magnetic flux in the inductors $L_1$ to $L_{24}$ due to the permanent magnet M is balanced. On the other hand, if the magnetic balance condition is broken, that is, the two groups of wires in the coils 24 and 25 are inversely biased relative to each other by the magnetic field of the permanent magnet M, the inductance of the first group 24 increases, while the inductance of the second group 25 decreases, or vice versa. Therefore, the differential level between the wires 21 and 23 is not zero, but has some level other than zero relating to the unbalance condition of the magnetic flux. Hence, the balance condition of the circuit is broken by the movement of the permanent magnet M and, therefore, the output level $E_{out}$ has some amplitude related to the displacement of the permanent magnet M. The plurality of radially-located inductors is used in the present embodiment because the effect of the earth's magnetism on each inductor is cancelled by the others. Therefore, the magnetic flux sensor is not influenced by the earth's magnetism and/or by external magnetic noise.

In operation, the diaphragm 3 is placed on the surface of the chest wall of a body to be examined, so that the diaphragm vibrates, reflecting the vibration of the body surface. The acoustic signal due to the vibration of the diaphragm 3 is transmitted to the ears of a doctor through the space 5 and the tubes 8 and 4.

Simultaneoulsy, the vibration of the diaphragm 3 vibrates the magnet M. The displacement of the vibration of the magnet M causes the generation of inductance variation in the inductors 12, i.e. the first group 24 and the second group 25. In other words, the balanced condition between the first group 24 and the second group 25 is broken, and the differential voltage $E_{out}$ obtained at the output of the processor 6 is proportional to the variation of the inductors coupled thereto through the sensor cable 7. The output signal $E_{out}$ can be displayed visually on a screen and/or the waveform can be printed on a paper.

Next, some experimental results are described. Fig. 5 shows a curve of output voltage $E_{out}$ when an external constant magnetic field is applied roughly perpendicular to the plane of the holder 11 (Fig.2). The horizontal axis shows the external magnetic field in Oersteds and the vertical axis shows the output voltage $E_{out}$ of the processor 6 in mV. As shown in Fig. 5, the output voltage $E_{out}$ does not change even when an external magnetic field up to $\pm 2$ Oe is applied.

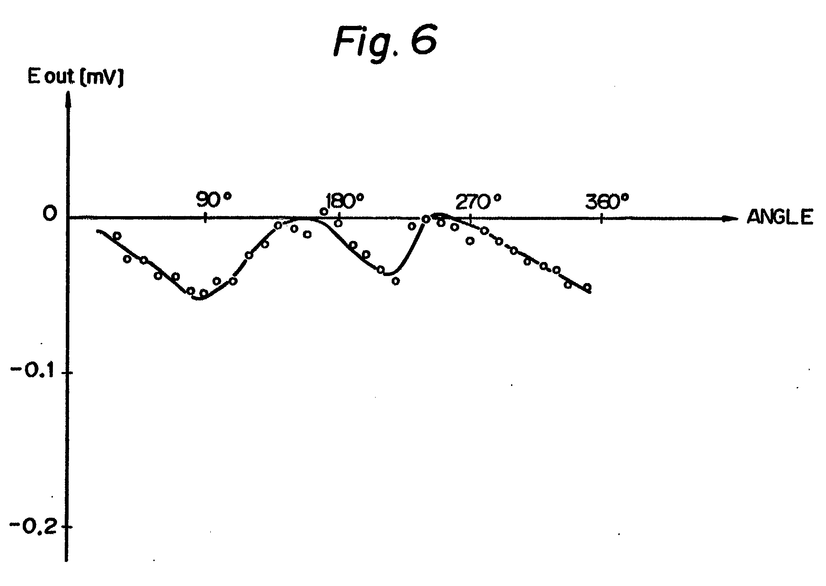

Fig. 6 shows a curve of the output voltage $E_{out}$ when the present sensor was rotated in a research laboratory in which many electric devices generated magnetic noise. In obtaining the curve of Fig. 6, the length of each coil $L_1$ to $L_{24}$ was 3 mm, the number of turns of each coil was 20, the source voltage E was 2,8 volt, and the sensor was not shielded. The horizontal axis in Fig. 6 shows the angle of rotation of the sensor, and the vertical axis shows the output voltage $E_{out}$. As shown in Fig. 6, the fluctuation of the output voltage $E_{out}$ was less than 0.06 mV when the sensor was rotated by 360°. Since the normal output voltage $E_{out}$ when the magnet M vibrates is around ±150mV, the fluctuation by 0.06mV was only 0.04%, which is very small.

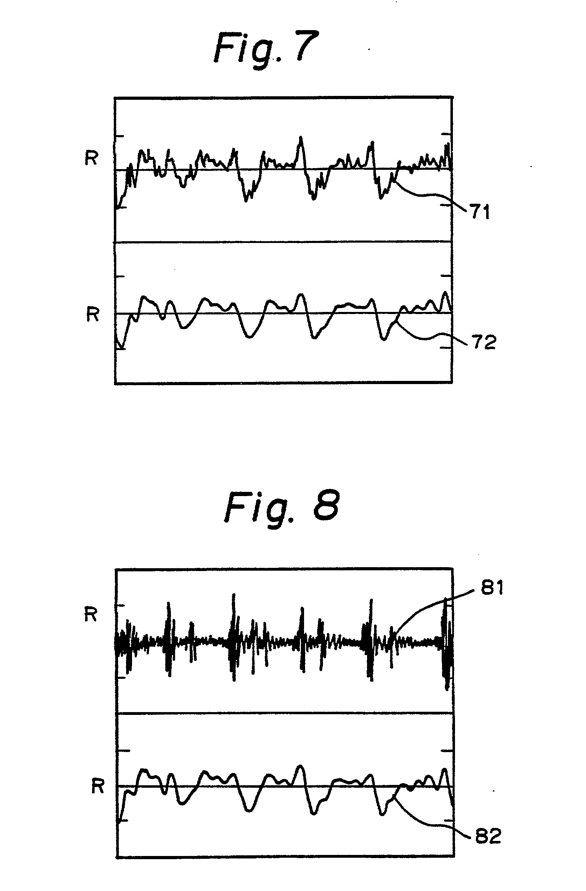

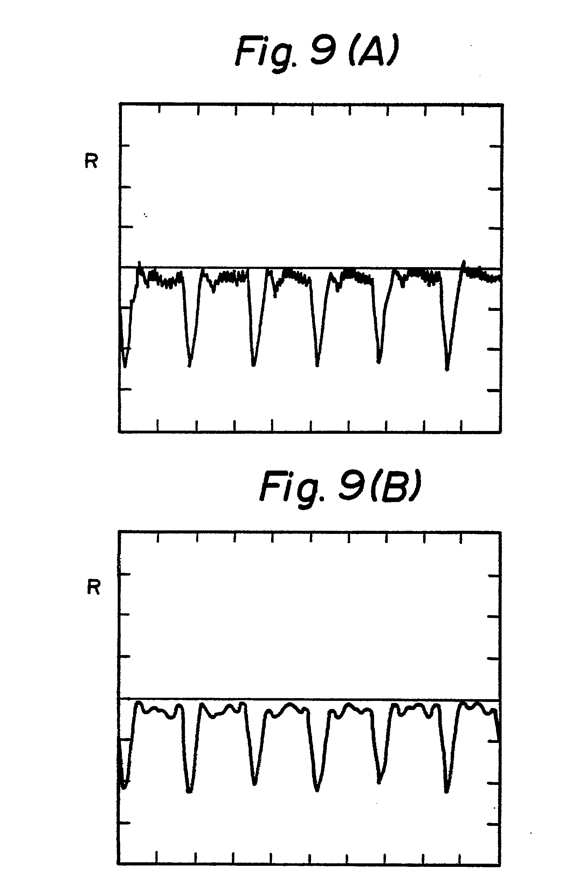

Some cardiograms obtained by using the present stethoscope will now be described, with reference to Figs. 7, 8 and 9. A curve 71 in Fig. 7 shows the total vibration wave obtained when the sensor was placed on the chest wall of a human body, and a curve 72 shows the heart beat wave obtained by deriving the component up to 20Hz of the curve 71. A curve 81 in Fig. 8 is a phonocardiogram which was obtained by using the 20-600Hz bandpass filter, and a curve 82 is the same as the curve 72 for comparing the phase relationship between the curves 81 and 82. Fig. 9A shows a curve of a wave produced by an artery in an elbow, and Fig. 9B is the result of low-pass filtering of the curve of Fig. 9A.

It should be appreciated that the above curves 71 to 82 are free from external noise caused by the earth's magnetism and/or caused by electrical devices, and that the curves are therefore very accurate and reliable sensor indicates the actual movement of a heart, while a prior cardiograph indicates only the electrical drive signal of the heart.

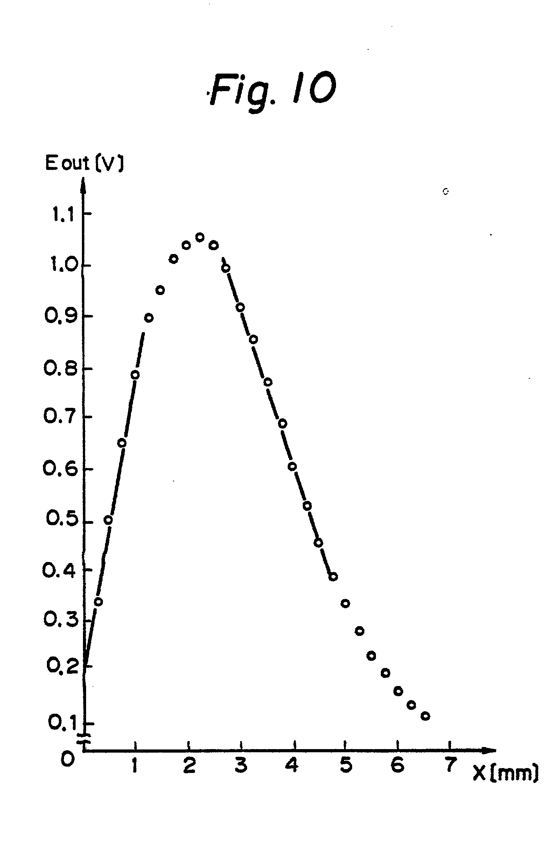

Fig. 10 is a curve showing the relationship between the displacement of the magnet M and the output voltage $E_{out}$. As shown in Fig. 10, there exists a linear portion in the curve, and it is preferable to use the sensor over this linear portion. In the present embodiment it is preferable that the sensor be used in the condition that X is between 3 mm and 4.5 mm, where X is the distance between the end of the magnet M and the plane of the inductors $L_1$ to $L_{24}$. The distance X is adjustable by adjusting the screw attachment between the main body 2 and the diaphragm 3.

Modifications of the present invention are of course possible to those skilled in the art. For instance, although twelve inductors are included in each inductor group 24 and 25, an inductor group having a different number of inductors is possible. A magnetic yoke made of amorphous material may be mounted between the magnet M and the ends of the inductors. Such yoke may be located in the holes 13 and 14. The yoke would concentrate the magnetic path of the magnet M, and would decrease the influence of the lateral movement of the magnet M. Furthermore, another permanent magnet could be located so that the magnetic flux is parallel to the inductors This would increase the sensitivity of the sensor.

As described above in detail, the present stethoscope provides not only an acoustic output but also an electrical output, which may be visually displayed and/or printed on a paper. Therefore, data relating to auscultation by an expert doctor is obtained on a real time basis. Therefore, the present stethoscope may be used as a training apparatus for new doctors.

When the present apparatus is used as a cardiograph, it is more powerful than a conventional type, because the present apparatus indicates the actual mechanical movement of the heart, whilst the conventional electrocardiograph provides merely an indication of the electrical drive signal to the heart. Conventional mechanocardiograph sensors such as microphones and acceleration transducers are rather difficult to use.

As the present inductor group has many radially located inductors which are connected in series alternately, external magnetic noise is cancelled in the inductors. Therefore, the present stethoscope can operate accurately, with high operational reliability, even if the sensor is not protected by a magnetic shield.

**Claims**

1. A displacement sensor, having a diaphragm (3) which is to be placed on a moving body; a magnetic pole (M) movable with the diaphragm; a plurality of inductors (24, 25, $L_1$ to $L_{24}$) located radially around the magnetic pole (M) on a plane which is perpendicular to the direction of movement of the diaphragm (3) to provide an output signal in dependence upon the position of the magnetic pole, the inductors ($L_1$ to $L_{24}$) being divided into two groups (24, 25) of alternate inductors with the inductors in each group being connected in series; and a processor (6) for processing the output signal of the inductors (24, 25).

2. A displacement sensor according to claim 1, having a hollow tube (8, 4) for acoustically coupling the diaphragm (3) with an ear of a user.

3. A displacement sensor according to claim 1 or 2, in which each of the inductors ($L_1$ to $L_{24}$) comprises a linear magnetic core (AF) made of amorphous material and a coil encircling the core.

4. A displacement sensor according to any preceding claim, in which the magnetic pole is a permanent magnet.

5. A displacement sensor according to any preceding claim, in which the processor (6) comprises an astable multivibrator coupled to the inductance means (24, 25).

6. A displacement sensor according to claim 5, in which the multivibrator oscillates at a frequency f and comprises a pair of transistors ($Tr_1$, $Tr_2$); a pair of parallel circuits of a capacitor ($C_B$) and a resistor ($R_B$) coupled between the base of a respective one of the transistors and the collector of the other transistor, the collector of each transistor being coupled to the inductance means (24, 25).

7. A displacement sensor according to claim 6, also including a balancing circuit (VR, $R_L$) bridging the emitters of the transistors ($Tr_1$, $Tr_2$), and a filter (ACF) coupled across the emitters to provide an output signal of the processor (6).

8. A displacement sensor according to claim 6 or claim 7, wherein the frequency f lies in a range between 100kHz and 500kHz.

9. A displacement sensor according to claim 7, in which the filter is a low-pass filter.

10. A displacement sensor according to any preceding claim, in which each of the two groups of inductors (24, 25) comprises twelve series-connected inductors.

11. A displacement sensor according to any preceding claim, in which the distance between the diaphragm (3) and the inductance means (24, 25) is adjustable.

## Patentansprüche

1. Verschiebungssensor mit einer Membran (3), die auf einem sichbewegenden Körper anzuordnen ist; einem zusammen mit der Membran bewegbaren Magnetpol (M); mehreren Induktionsmitteln (24, 25, $L_1$ bis $L_{24}$), die radial um den Magnetpol (M) in einer zur Bewegungsrichtung der Membran (3) senkrechten Ebene angeordnet sind, um ein Ausgangssignal in Abhängigkeit von der Lage des Magnetpols zu bilden, wobei die Induktionsmittel ($L_1$ bis $L_{24}$) in zwei Grupen (24, 25) einander abwechselnder Induktionsmittel unterteilt und in jeder Gruppe in Reihe geschaltet sind; und einem Prozessor (6) zum Verarbeiten des Ausgangssignals der Induktionsmittel (24, 25).

2. Verschiebungssensor nach Anspruch 1 mit einem hohlen Rohr (8, 4) zum akustischen Ankoppeln der Membran (3) an ein Ohr eines Benutzers.

3. Verschiebungssensor nach Anspruch 1 oder 2, bei dem jedes Induktionsmittel ($L_1$ bis $L_{24}$) einen geradlinigen Magnetkern (AF) aus amorphem Material und eine den Kern umgebende Spule aufweist.

4. Verschiebungssensor nach einem der vorstehenden Anspüche, bei dem der Magnetpol ein Dauermagnet ist.

5. Verschiebungssensor nach einem der vorstehenden Ansprüche, bei dem der Prozessor (6) einen mit den Induktionsmitteln (24, 25) verbundenen astabilen Multivibrator aufweist.

6. Verschiebungssensor nach Anspruch 5, bei dem der Multivibrator mit einer Frequenz f schwingt und zwei Transistoren (Tr1, Tr2) sowie zwei parallele Schaltungen aus einem Kondensator ($C_B$) und einem ohmschen Widerstand ($R_B$) aufweist, die zwischen der Basis jeweils eines der Transistoren und dem Kollektor des anderen Transistors angeschlossen sind, wobei der Kollektor jedes Transistors mit den Induktionsmitteln (24, 25) verbunden ist.

7. Verschiebungssensor nach Anspruch 6, der ferner eine Abgleichschaltung (VR, $R_L$), die die Emitter der Transistoren (Tr1, Tr2) überbrückt, und ein Filter (ACF) aufweist, das zwischen den Emittern angeschlossen ist, um ein Ausgangssignal des Prozessors (6) zu erzeugen.

8. Verschiebungssensor nach Anspruch 6 oder Anspruch 7, bei dem die Frequenz f in einem Bereich zwischen 100 kHz und 500 kHz liegt.

9. Verschiebungssensor nach Anspruch 7, bei dem das Filter ein Tiefpaßfilter ist.

10. Verschiebungssensor nach einem der vorstehenden Ansprüche, bei dem jede der beiden Gruppen von Induktionsmittteln (24, 25) zwölf in Reihe geschaltete Induktionsmittel aufweist.

11. Verschiebungssensor nach einem der vorstehenden Ansprüche, bei dem der Abstand zwischen der Membran (3) und den Induktionsmitteln (24, 25) einstellbar ist.

## Revendications

1. Capteur de déplacement comprenant un diaphragme (3) qui doit être placé sur un corps mobile; un pôle magnétique (M) déplaçable avec le diaphragme; une pluralité d'inductances (24, 25, $L_1$ à $L_{24}$) disposées radialement autour du pôle magnétique (M) sur un plan perpendiculaire au sens du déplacement du diaphragme (3) pour fournir un signal de sortie en fonction de la position du pôle magnétique, les inductances ($L_1$ à $L_{24}$) étant divisées en deux groupes (24, 25) d'inductances alternées, les inductances de chaque groupe étant connectées en série; et un processeur (6) pour traiter le signal de sortie des inductances (24, 25).

2. Capteur de déplacement selon la revendication 1, comprenant un tube creux (8, 4) pour coupler acoustiquement le diphragme (3) à l'oreille d'un utilisateur.

3. Capteur de déplacement selon la revendication 1 ou 2, dans lequel chacune des inductances ($L_1$ à $L_{24}$) comprend un noyau magnétique linéaire (AF) constitué d'un matériau amorphe et un enroulement entourant le noyau.

4. Capteur de déplacement selon l'une quelconque des revendications précédentes dans lequel le pôle magnétique est un aimant permanent.

5. Capteur de déplacement selon l'une quelconque des revendications précédentes dans lequel le processeur (6) comprend un multivibrateur astable couplé aux moyens d'inductances (24, 25).

6. Capteur de déplacement selon la revendication 5, dans lequel le multivibrateur oscille à une fréquence f et comprend une paire de transistors (Tr1, Tr2), une paire de circuits parallèles d'un condensateur ($C_B$) et d'une résistance ($R_B$) couplée entre la base de l'un respectif des transistors et le collecteur de l'autre transistor, le collecteur de chaque transistor étant couplé aux moyens d'inductances (24, 25).

7. Capteur de déplacement selon la revendication 6, comprenant en outre un circuit d'équilibrage (VR, $R_L$) formant pont entre les émetteurs des transistors (Tr1, Tr2) et un filtre (ACF) couplé aux bornes des émetteurs pour fournir un signal de sortie du processeur (6).

8. Capteur de déplacement selon la revendication 6 ou 7, dans lequel la fréquence f se trouve dans une gamme comprise entre 100 kHz et 500 kHz.

9. Capteur de déplacement selon la revendication 7, dans lequel le filtre est un filtre passe-bas.

10. Capteur de déplacement selon l'une quelconque des revendications précédentes dans lequel chacun des deux groupes d'inductances (24, 25) comprend douze inductances connectées en série.

11. Capteur de déplacement selon l'une quelconque des revendications précédentes dans lequel la distance entre le diaphragme (3) et les moyens d'inductances (24, 25) est réglable.

# Fig. 1

# Fig. 4

## Fig. 2(A)

## Fig. 2(B)

# Fig. 3

EP 0 228 178 B1

Fig. 5

Fig. 6

EP 0 228 178 B1

# Fig. 7

# Fig. 8

# Fig. 9 (A)

# Fig. 9(B)

Fig. 10